# EUROPÄISCHE PATENTSCHRIFT

(11) **EP 3 865 587 B1**
(45) Veröffentlichungstag und Bekanntmachung des Hinweises auf die Patenterteilung: **05.08.2026**
(21) Anmeldenummer: 21156691.4
(22) Anmeldetag: 11.02.2021
(51) Int. Cl.: C12Q 1/682, C12Q 1/6841

(54) **SONDENKOMPLEX ZUM SPEZIFISCHEN NACHWEIS VON WENIGSTENS EINER SUBSTANZ, KORRESPONDIERENDES VERFAHREN UND VERWENDUNG**
SAMPLING STRUCTURE FOR THE SPECIFIC DETECTION OF AT LEAST ONE SUBSTANCE, METHOD AND USE THEREOF
COMPLEXE DE SONDE PERMETTANT DE DÉCELER DE MANIÈRE SPÉCIFIQUE LA PRÉSENCE D'AU MOINS UNE SUBSTANCE, PROCÉDÉ CORRESPONDANT ET UTILISATION

(30) Priorität: 14.02.2020 DE 102020103966
(43) Veröffentlichungstag der Anmeldung: 18.08.2021
(73) Patentinhaber: Testo bioAnalytics GmbH, 79822 Titisee-Neustadt (DE)
(72) Erfinder: Riemer, Joel, 79874 Breitnau (DE)
(74) Vertreter: Mertzlufft-Paufler, Cornelius

(56) Entgegenhaltungen:
- WO-A1-2017/189525
- WO-A1-2018/194755
- US-A1- 2017 101 672
- HARRY M. T. CHOI ET AL: "Third-generation in situ hybridization chain reaction: multiplexed, quantitative, sensitive, versatile, robust", DEVELOPMENT, vol. 145, no. 12, 15 June 2018 (2018-06-15), GB, XP055590305, ISSN: 0950-1991, DOI: 10.1242/dev.165753
- ALEXANDRE BOUTORINE ET AL: "Fluorescent Probes for Nucleic Acid Visualization in Fixed and Live Cells", MOLECULES, vol. 18, no. 12, 1 January 2013 (2013-01-01), pages 15357 - 15397, XP055187487, DOI: 10.3390/molecules181215357
- YAMAGUCHI TSUYOSHI ET AL: "In situ DNA-hybridization chain reaction (HCR): a facilitated in situ HCR system for the detection of environmental microorganisms : In situ DNA-HCR for environmental microorganisms", ENVIRONMENTAL MICROBIOLOGY, vol. 17, no. 7, 11 February 2015 (2015-02-11), GB, pages 2532 - 2541, XP055818908, ISSN: 1462-2912, DOI: 10.1111/1462-2920.12745
- ZHAO DAN ET AL: "Nucleic acid circuits for cell imaging: From the test tube to the cell", TRAC TRENDS IN ANALYTICAL CHEMISTRY, ELSEVIER, AMSTERDAM, NL, vol. 122, 1 November 2019 (2019-11-01), XP086023664, ISSN: 0165-9936, [retrieved on 20191101], DOI: 10.1016/J.TRAC.2019.115706

## Beschreibung

Die Erfindung betrifft einen Sondenkomplex zum spezifischen Nachweis von wenigstens einer Substanz, wobei der Sondenkomplex an ein Ziel-Segment der Substanz spezifisch anbindet und wobei durch eine Interaktion mit wenigstens einer detektierbaren Markierung ein vorzugsweise optisch nachweisbares Signal auslösbar ist.

Zum spezifischen Nachweis von Nukleinsäuren, d.h. DNA- und/oder RNA-Molekülen in einzelnen Zellen sind beispielsweise Verfahren wie In-Situ-Hybridisierung (ISH) bekannt. Dabei werden kurze synthetische Nukleinsäure-Sonden eingesetzt, welche über komplementäre Basenpaarungen an die nachzuweisende Zielsequenz binden. Eine Variante der ISH-Technologie, bei welcher die Nukleinsäure-Sonden fluoreszenzmarkiert sind, ist die Fluoreszenz-In-Situ-Hybridisierung (FISH).

Mit molekularbiologischen Verfahren, wie beispielsweise Fluoreszenz-In-Situ-Hybridisierung, kann die Nukleinsäure eines Mikroorganismus in wenigen Stunden oder Minuten nachgewiesen werden. Bei einem solchen vorbekannten Nachweisverfahren können jedoch typischerweise nur Ziel-Substanzen mit hoher Kopienzahl, wie beispielsweise rRNA, verwendet werden, um ausreichend hohe Signalstärke zu erreichen. Dabei kann auch eine Differenzierung zwischen lebenden und toten Mikroorganismen stattfinden, die allerdings nur innerhalb gleicher Gattung bzw. Familie möglich ist, da unterschiedliche Mikroorganismen unterschiedliche rRNA-Gehalte aufweisen können und in Abhängigkeit davon große Varianzen in den resultierenden Signalstärken auftreten können. Außerdem kann rRNA unter Umständen auch von toten Mikroorganismen nachgewiesen werden. Es ist also wünschenswert, Sequenzen mit kürzerer Halbwertszeit als rRNA, jedoch unter Umständen geringerer Kopienzahl (wie beispielsweise mRNA-Sequenzen) detektieren zu können, um so eine bessere lebend/tot-Unterscheidung zu ermöglichen. Ferner ist es wünschenswert, weitere und unter Umständen spezifischere Sequenzen (beispielsweise Art-, Serotyp-, Teilungs- oder Pathogenitäts-spezifische Sequenzen), die in geringerer Kopienzahl vorliegen können (wie beispielsweise mRNA- oder DNA-Sequenzen) zu detektieren.

Des Weiteren kennt man bereits enzymbasierte Verfahren, auf welche für die Unterscheidung von beispielsweise Leben/Tot-Stadium der nachzuweisenden Mikroorganismen zurückgegriffen wird. Typischerweise ist dabei eine Enzymaktivität als "lebend"-Indikator detektierbar, obwohl die nachzuweisenden Mikroorganismen nicht mehr teilungsfähig sind. Dadurch kann eine zuverlässige Analytik erschwert werden. Die Veröffentlichung von Harry M. T. Choi et al., 2018, offenbart ein Hybridisierungsverfahren mit zwei alternierende Sondentypen das zur Signalverstärkung eingesetzt wird.

Von Vorteil ist dabei, dass eine einfache und kostengünstige Analytik erreichbar ist, die eine Bestimmung unterschiedlicher Ziel-Sequenzen innerhalb eines Organismus in einem Verfahren gewährleisten kann.

Vor diesem Hintergrund ist es Aufgabe der vorliegenden Erfindung, einen Sondenkomplex bereitzustellen, der eine spezifische Detektion von Ziel-Substanzen, beispielsweise Nukleinsäure-Sequenzen oder Aminosäuren-Sequenzen, in einem Verfahren ermöglicht, das kostengünstig und außerhalb von Laborumgebungen durchführbar ist.

Erfindungsgemäß wird diese Aufgabe durch die Merkmale von Anspruch 1 gelöst.

Insbesondere wird somit erfindungsgemäß zur Lösung der genannten Aufgabe bei einem Sondenkomplex der eingangs beschriebenen Art vorgeschlagen, dass in dem Sondenkomplex wenigstens drei Sondentypen vorhanden sind, wobei der Sondenkomplex an ein Ziel-Segment der wenigstens einen Substanz spezifisch anbindet, wobei durch eine Interaktion mit wenigstens einer detektierbaren Markierung ein vorzugsweise optisch nachweisbares Signal auslösbar ist. Der erfindungsgemäße Sondenkomplex ist so ausgebildet, dass er wenigstens einen Sondentyp als eine Primärsonde mit einem zielspezifischen Segment aufweist, das zu dem Ziel-Segment der Substanz komplementär ist, und wenigstens einen weiteren Sondentyp als eine Sekundärsonde mit einem zielspezifischen Segment aufweist, das zu der wenigstens einen Primärsonde komplementär ist, wobei die zielspezifischen Segmente der Sondentypen so aufeinander abgestimmt sind, dass eine Kette von wenigstens drei, insbesondere beliebig vielen, Sonden bildbar ist, sodass eine Kettenlänge gestaltet ist. Somit kann eine Kettenlänge flexibel gestaltet sein, insbesondere, wenn die Kette von Sonden verzweigt oder unverzweigt ausgebildet ist, wie nachfolgend beschrieben und/oder beansprucht.

Somit kann die Detektierbarkeit von Substanzen signifikant verbessert werden, da durch die Bildung einer Kette von markierten Sonden eine Signalverstärkung bei optischem Detektieren ermöglicht werden kann. Durch die Verwendung von wenigstens drei Sondentypen in dem Sondenkomplex kann eine stabile Kette von Sonden gebildet werden, die eine Signalverstärkung bei optischem Nachweis gewährleistenkann. Vorteilhaft ist dabei, dass der Prozess der Markierung vereinheitlicht werden kann, da immer die gleiche Sequenz als Sekundärsonde verwendbar ist und lediglich die Primärsonde an die Ziel-Substanz angepasst werden soll. Der Begriff "komplementär" kann sich im Sinne der Erfindung auf gegensätzliche, aber sich ergänzende Eigenschaften eines Objekts beziehen. Dies könnte unter anderem auch eine Komplementärbindung aufgrund der Bildung von Antigen-Antikörper-Komplexen sowie andere komplementäre Strukturen der Reaktionspartnern umfassen, die über nicht-kovalente Bindungen miteinander interagieren, wie es beispielsweise bei DNA-Doppelhelix der Fall ist.

Nachfolgend werden vorteilhafte Ausgestaltungen der Erfindung beschrieben, die allein oder in Kombination mit den Merkmalen anderer Ausgestaltungen optional zusammen mit den Merkmalen nach Anspruch 1 kombiniert werden können.

Eine vorteilhafte Ausführungsform gemäß der Erfindung sieht vor, dass der wenigstens eine Sondentyp eine Nukleinsäure oder ein Antikörper oder ein Peptid ist. Somit sind die Erkennung und damit die Detektion einer Substanz durch die Bindung über komplementäre Basenpaare der jeweils als Kette ausgebildeten zielspezifischen Segmente der Nukleinsäure-Sonden oder durch die Bindung eines Antigens an einen Antikörper erreichbar.

Eine vorteilhafte Ausführungsform gemäß der Erfindung sieht vor, dass in dem Sondenkomplex wenigstens vier Sondentypen vorhanden sind. Dadurch kann eine stabile Kette von Sonden gebildet werden, die eine Signalverstärkung bei optischem Nachweis gewährleisten kann.

Eine vorteilhafte Ausführungsform gemäß der Erfindung sieht vor, dass die Kette von Sonden verzweigt oder unverzweigt ausgebildet ist. Dadurch ist eine flexible Gestaltung in der Kettenlänge und/oder Kettenstruktur des Sondenkomplexes erreichbar, die an die detektierbare Substanz angepasst bzw. optimiert werden kann.

Eine vorteilhafte Ausführungsform gemäß der Erfindung sieht vor, dass in dem Sondenkomplex wenigstens ein weiterer Sondentyp als eine Tertiärsonde mit einem zielspezifischen Segment ausgebildet ist, das zu der wenigstens einen Sekundärsonde komplementär ist. Insbesondere hierbei vorgesehen sein, dass der Sondenkomplex zusätzlich mit wenigstens einem weiteren Sondentyp als eine Quartärsonde mit einem zielspezifischen Segment, das zu der wenigstens einen Tertiärsonde komplementär ist, ausgebildet ist. Alternativ oder zusätzlich ist in wenigstens einer Quartiärsonde ein zielspezifisches Segment ausgebildet, das zu der wenigstens einen Sekundärsonde komplementär ist. Auf diese Weise kann eine verzweigte oder unverzweigte Kettenstruktur zwischen dem Ziel-Segment der Substanz und dem Sondenkomplex ausgebildet werden. Somit sind eine Signalverstärkung und eine höhere Fluoreszenzintensität erreichbar, da eine Bindung mehrerer Sekundär-, Tertiär- und/oder Quartärsonden an eine Primärsonde ermöglicht werden kann und mehrere Farbmarkierungen pro Substanz genutzt werden können.

Eine vorteilhafte Ausführungsform gemäß der Erfindung sieht vor, dass die Sondentypen jeweils als lineare Sonden ausgebildet sind. Beispielsweise sind das monoProbes, dopeProbes, tetraProbes und multiProbes. Alternativ oder zusätzlich können die Sondentypen jeweils als Sonden mit Sekundärstruktur, vorzugsweise eine Haarnadel-Sonde, ausgebildet sein. Beispielsweise sind das Molecular Beacons und Scorpions Sonden. Dadurch sind eine höhere Fluoreszenzintensität sowie ein besseres Signal-RauschVerhältnis erreichbar, was insbesondere für eine automatisierte Anwendung vorteilhaft ist.

Eine vorteilhafte Ausführungsform gemäß der Erfindung sieht vor, dass der Sondenkomplex wenigstens eine detektierbare Markierung aufweist. Es kann vorgesehen sein, dass in dem Sondenkomplex jeder Sondentyp die wenigstens eine detektierbare Markierung aufweisen kann. Insbesondere kann vorgesehen sein, dass die wenigstens eine detektierbare Markierung an der Primärsonde ausgebildet ist. Alternativ oder zusätzlich kann in dem Sondenkomplex eine Primärsonde frei von Markierungen ausgebildet sein und wenigstens ein weiterer, insbesondere als eine Sekundär-, Tertiär- und/oder Quartärsonde ausgebildeter, Sondentyp die wenigstens eine detektierbare Markierung aufweisen. Die detektierbare Markierung kann beispielsweise ein Enzymmarker, Affinitätsmarker und/oder ein Farbstoff sein. Der Farbstoff kann beispielsweise ein Fluoreszenzfarbstoff sein. Somit ist eine optische Detektion erreichbar. Der Affinitätsmarker kann beispielsweise Affinitätsbindungspaare Biotin-Streptavidin oder Antigen-Antikörper umfassen.

Eine vorteilhafte Ausführungsform gemäß der Erfindung sieht vor, dass das Ziel-Segment der Substanz wenigstens eine DNA- und/oder RNA-Sequenz umfasst. Es kann insbesondere vorgesehen sein, dass das Ziel-Segment der Substanz mRNA-Sequenz umfasst. Somit kann ein spezifischer Nachweis von Substanzen auf DNA- und/oder RNA-Ebene ermöglicht werden. Vorteilhaft dabei ist, dass nicht nur Ziel-Substanzen mit hoher Kopienzahl, wie beispielsweise rRNA, sondern auch andere RNA mit geringer Kopienzahl, insbesondere mRNA- oder tRNA-, oder DNA-Sequenzen mit geringer Kopienzahl, wie beispielsweise Art-, Serotyp-, Teilungs- oder Pathogenitäts-spezifische Sequenzen, innerhalb eines Organismus detektierbar sind. Die Erfindung macht sich zunutze, dass insbesondere Haushaltsgene (Housekeeping-Gene), wie beispielsweise mRNA, detektierbar sind, die in teilungsfähigen Zellen ständig exprimiert werden, da sie zur Aufrechterhaltung grundlegender Zellfunktionen erforderlich sind.

Alternativ oder zusätzlich kann das Ziel-Segment der Substanz eine Aminosäuren-Sequenz umfassen. Somit kann ein spezifischer Nachweis von Substanzen auf Peptid- und/oder Protein-Ebene ermöglicht werden.

Eine vorteilhafte Ausführungsform gemäß der Erfindung sieht vor, dass die Substanz eine Nukleinsäure umfasst. Alternativ oder zusätzlich kann die Substanz ein Protein, insbesondere ein Antikörper, umfassen. Dadurch sind die Erkennung und damit die Detektion einer Substanz durch die Bindung eines Antigens an einen Antikörper erreichbar. Vorteilhaft ist dabei, dass die hohe Spezifität und Bindungsstärke der Bindung zwischen Antigenen und Antikörpern genutzt werden kann. Alternativ oder zusätzlich kann die Substanz ein Virus und/oder eine niedermolekulare Verbindung, insbesondere ein Hormon, Toxin und/oder Pestizid, umfassen.

Ein weiterer Gegenstand der Erfindung ist ein Verfahren zur Durchführung von Sonden-Kettenreaktion, umfassend die folgenden Schritte: a) Inkontaktbringen einer vorzugsweise in einer Probe enthaltenen Substanz mit wenigstens einem Sondenkomplex, insbesondere nach einem der Ansprüche 1 bis 9; b) Durchführung eines ersten Kreislaufs einer Sonden-Kettenreaktion, wobei wenigstens eine Primärsonde an wenigstens ein Ziel-Segment der Substanz bindet und wenigstens eine Sekundärsonde an die wenigstens eine Primärsonde bindet, wobei wenigstens eine Tertiärsonde an die wenigstens eine Sekundärsonde bindet und wenigstens eine Quartärsonde an die wenigstens eine Tertiärsonde und/oder an die wenigstens eine Sekundärsonde bindet; c) optional Durchführung eines zweiten Kreislaufs einer Sonden-Kettenreaktion, wobei wenigstens eine weitere Sonde an wenigstens eine der Sekundärsonde, Tertiärsonde und/oder Quartärsonde bindet; und d) vorzugsweise optisches Detektieren der in den einzelnen Substanzen erzeugten Signalen.

Eine vorteilhafte Ausführungsform gemäß der Erfindung sieht vor, dass die Durchführung der Sonden-Kettenreaktion oder der Sonden-Kettenreaktionen beispielsweise generell mittels eines Detektionsverfahrens erfolgen kann, das auf einer Farbreaktion basiert. Insbesondere kann die Durchführung der Sonden-Kettenreaktion oder der Sonden-Kettenreaktionen unter Verwendung des Sondenkomplexes mittels Fluoreszenz-In-Situ-Hybridisierung (FISH) erfolgen. Alternativ oder zusätzlich kann die Durchführung der Sonden-Kettenreaktion oder der Sonden-Kettenreaktionen unter Verwendung des Sondenkomplexes mittels Immunassays erfolgen. Beispielsweise ist die Durchführung der Sonden-Kettenreaktion oder der Sonden-Kettenreaktionen mittels Enzym-basierter Assays, vorzugsweise Enzyme-linked Immunosorbent Assays (ELISA), ausführbar.

Eine bevorzugte Anwendung sieht ein Testkit zur Durchführung des Verfahrens nach einem der auf ein Verfahren gerichteten Ansprüche vor, umfassend wenigstens einen Sondenkomplex nach einem der auf ein Sondenkomplex gerichteten Ansprüche und vorzugsweise wenigstens ein Reagenz mit Enzym.

Eine bevorzugte Anwendung sieht eine Verwendung des Sondenkomplexes nach einem der auf einen Sondenkomplex gerichteten Ansprüche und/oder eines Verfahrens nach einem der auf eine Verfahren gerichteten Ansprüche und/oder eines Testkits nach einem auf ein Testkit gerichteten Anspruch zum spezifischen Nachweis von Mikroorganismen und/oder deren Eigenschaften.

Eine bevorzugte Anwendung sieht eine Verwendung des Sondenkomplexes nach einem auf eine Verwendung gerichteten Anspruch zur Signalverstärkung bei optischem Detektieren mittels Farbumschlag, insbesondere mittels Fluoreszenz-In-Situ-Hybridisierung (FISH) und/oder Immunassay, bei dem wenigstens ein Sondentyp vorhanden ist, wobei wenigstens zwei Sondentypen so aneinander binden, dass eine Kette von Sonden gebildet werden kann. Beispielsweise kann eine Verwendung des Sondenkomplexes nach einem auf eine Verwendung gerichteten Anspruch zur Signalverstärkung bei optischem Detektieren mittels Enzym-basierter Assays, vorzugsweise Enzyme-linked Immunosorbent Assays (ELISA) erfolgen. Eine Verwendung des Sondenkomplexes nach einem auf eine Verwendung gerichteten Anspruch kann beispielsweise generell zur Signalverstärkung bei optischem Detektieren mittels eines Detektionsverfahrens erfolgen, das auf einer Farbreaktion basiert.

Es kann vorgesehen sein, dass das Verfahren mit einem fluidischen Kanalsystem ausführbar ist. Beispielsweise kann ein fluidisches Kanalsystem in Form eines Probenträgers ausgebildet sein. Der Probenträger kann insbesondere wenigstens eine Kavität mit wenigstens einem Sondenkomplex und wenigstens eine Substanz umfassen. Alternativ oder zusätzlich kann der Probenträger mit Mitteln zur optischen Zählung von markierten Mikroorganismen vorgesehen sein. Der Probenträger kann als ein scheibenförmiger Probenträger ausgebildet sein. Beispielsweise kann der Probenträger als ein flacher Probenträger ausgebildet sein. Es sind auch alternativ rechteckförmige Probenträger wie bei einer Chipkarte oder segmentförmige Probenträger wie bei einem Pizzastück bildbar.

Durch die Ausführung des erfindungsgemäßen Verfahrens mit einem fluidischen Kanalsystem vorzugsweise in Form eines Probenträgers ist erreichbar, dass ein spezifischer Nachweis von wenigstens einer Substanz, und somit ein spezifischer Nachweis von Mikroorganismen, in unterschiedlichen Applikationsfeldern ermöglicht werden kann. Beispielsweise kann das erfindungsgemäße Verfahren zur mikrobiologischen Lebensmittelanalyse, Hygienekontrolle, klinischen und biotechnologischen Anwendungen sowie Umweltanalyse zum Einsatz kommen.

Die Erfindung wird nun anhand von Ausführungsbeispielen näher beschrieben, ist aber nicht auf diese Ausführungsbeispiele beschränkt. Weitere Ausführungsbeispiele ergeben sich durch Kombination der Merkmale einzelner oder mehrerer Schutzansprüche untereinander und/oder mit einzelnen oder mehreren Merkmalen der Ausführungsbeispiele.

Es zeigt:

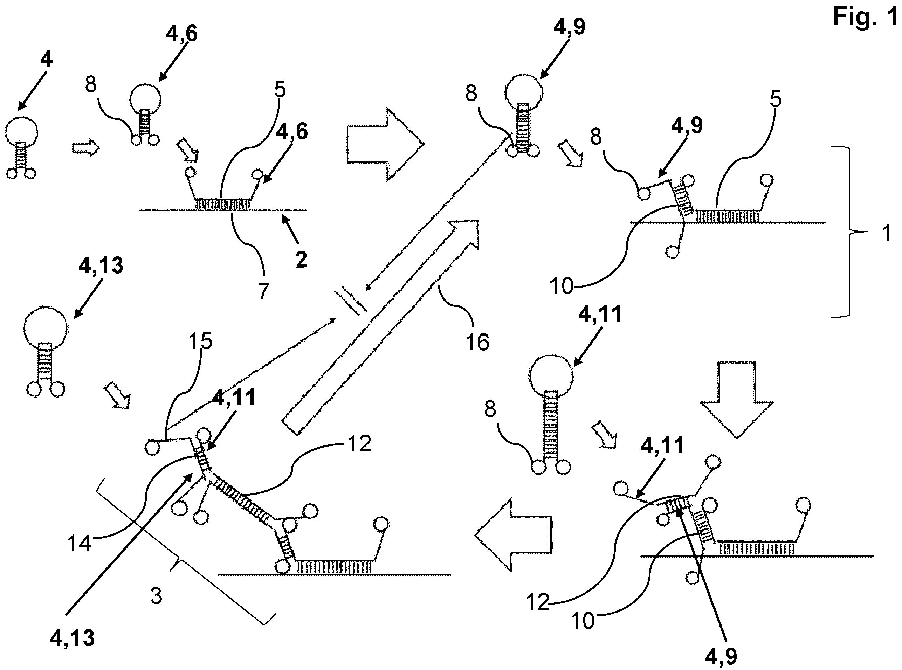

- Fig. 1: eine Ausführungsvariante eines erfindungsgemäßen Sondenkomplexes zum Ausführen eines erfindungsgemäßen Verfahrens zur Durchführung von Sonden-Kettenreaktion in einer schematischen Darstellung.

In der Figur 1 ist ein mögliches Ausführungsbeispiel eines erfindungsgemäßen Sondenkomplexes in vereinfachter schematischer Form dargestellt, wobei der erfindungsgemäße Sondenkomplex im Ganzen als 1 bezeichnet ist.

Der erfindungsgemäße Sondenkomplex 1 ist dazu ausgelegt, ein spezifisches Nachweisverfahren von wenigstens einer Substanz 2 vorzunehmen. Der Sondenkomplex 1 bildet dabei eine Kette 3 von wenigstens drei, insbesondere beliebig vielen, Sonden 4. Insbesondere bindet dabei der Sondenkomplex 1 über ein zielspezifisches Segment 5 einer Primärsonde 6 an ein vorzugsweise komplementäres Ziel-Segment 7 der zu detektierenden Substanz 2.

Um einen Nachweis vornehmen zu können, weist der Sondenkomplex 1 wenigstens eine detektierbare Markierung 8 auf. Hierbei kann es sich zum Beispiel um ein Enzymmarker, Affinitätsmarker und/oder ein Farbstoff handeln, die einen optischen Nachweis ermöglichen.

Der Sondenkomplex 1 weist wenigstens einen Sondentyp als eine Primärsonde 6 und wenigstens einen weiteren Sondentyp als eine Sekundärsonde 9 auf, die jeweils zielspezifische Segmente aufweisen, die so aufeinander abgestimmt sind, dass eine Kette 3 von wenigstens drei, insbesondere beliebig vielen, Sonden 4 bildbar ist. Der Begriff "Sonde" kann sich im Sinne der Erfindung auf eine Nukleinsäure oder ein Antikörper oder ein anderes Peptid beziehen.

Um eine Detektion einer spezifischen Anbindung des Sondenkomplexes 1 an eine Substanz 2 detektieren zu können, müssen die zu detektierende Substanz 2, die wenigstens eine Primärsonde 6 und die wenigstens eine Sekundärsonde 9 miteinander verbunden sein. Die Verbindung kann hierbei zum Beispiel über komplementäre Basenpaare der jeweils als Kette ausgebildeten zielspezifischen Segmente 5, 7 und 10 hergestellt sein.

Die wenigstens eine Primärsonde 6 weist das oben genannte zielspezifische Segment 5 auf, das an das Ziel-Segment 7 der Substanz 2 bindet. Die Primärsonde 6 kann zum Beispiel dazu ausgelegt sein, um einen Nachweis von RNA mit geringer Kopienzahl, insbesondere mRNA- oder tRNA-, und/oder DNA-Sequenzen mit geringer Kopienzahl, wie beispielsweise Art-, Serotyp-, Teilungs- oder Pathogenitäts-spezifische Sequenzen, spezifisch innerhalb eines Organismus zu ermöglichen. Alternativ oder zusätzlich kann ein Nachweis von Aminosäuren-Sequenzen, d.h. spezifischer Nachweis von Substanzen auf Peptid- und/oder Protein-Ebene, ermöglicht werden.

Die Figur 1 zeigt, dass die als Nukleinsäuren oder Antikörper ausgebildeten Sonden 4 jeweils die oben genannte wenigstens eine Markierung 8 aufweisen, durch welche ein detektierbares Signal erzeugbar ist. Die wenigstens eine Sekundärsonde 9 weist die Eigenschaften auf, dass sie ein zielspezifisches Segment 10 enthält, das zu der wenigstens einen Primärsonde 6 komplementär ist. Die Schmelztemperatur der Sekundärsonde 9 ist so gewählt, dass unter den gegebenen Assay-Bedingungen diese an das komplementäre Segment der Primärsonde 6 anbindet.

Diese Schmelztemperatur kann beispielsweise bei gleich oder höher als die für das Hybrid der Primärsonde mit dem Ziel-Segment 7 der Substanz 2 liegen.

Die Figur 1 zeigt, dass die wenigstens eine Tertiärsonde 11 so ausgebildet sein kann, dass sie ein zielspezifisches Segment 12 enthält, das zu der wenigstens einen Sekundärsonde 9 komplementär ist. Aus Figur 1 ist weiterhin ersichtlich, dass in dem Sondenkomplex 1 wenigstens ein weiterer Sondentyp als eine Quartärsonde 13 mit einem zielspezifischen Segment 14 ausgebildet sein kann, das zu der wenigstens einen Tertiärsonde 11 komplementär ist, und/oder mit einem zielspezifischen Segment 15, das zu der wenigstens einen Sekundärsonde 9 komplementär ist. Auf diese Weise ist eine verzweigte oder unverzweigte Kettenstruktur zwischen dem Ziel-Segment 7 der Substanz 2 und dem Sondenkomplex 1 bildbar.

Die Verwendung einer Kettenstruktur zwischen dem Ziel-Segment 7 der Substanz 2 und dem Sondenkomplex 1 ermöglicht eine Signalverstärkung und eine höhere Fluoreszenzintensität, da eine Bindung mehrerer Sekundär- Tertiär- und/oder Quartärsonden an eine Primärsonde 6 und eine Nutzung mehrerer Markierungen 8, insbesondere Farbmarkierungen, pro Substanz 2 erreichbar sind. Es ermöglicht zudem einen vereinfachten Nachweis von RNA-Sequenzen mit geringer Kopienzahl, insbesondere mRNA- oder tRNA-, und/oder DNA-Sequenzen mit geringer Kopienzahl, wie beispielsweise Art-, Serotyp-, Teilungs- oder Pathogenitäts-spezifische Sequenzen, spezifisch innerhalb eines Organismus.

In der Figur 1 ist eine Ausführungsvariante eines Sondenkomplexes 1 gezeigt, mit den Sonden 4, die jeweils als Haarnadel-Sonden ausgebildet sind. In einer weiteren, hier nicht gezeigten Ausführungsvariante kann der Sondenkomplex 1 so ausgebildet sein, dass wenigstens eine Sonde 4 als lineare Sonde ausgebildet ist und aufgrund ihrer Sequenzabfolge keine internen Sekundärstrukturen ausbilden kann.

Der hierin beschriebene und/oder beanspruchte Sondenkomplex 1 eignet sich somit insbesondere zur Verwendung in einem Verfahren zur Durchführung von Sonden-Kettenreaktion, insbesondere zur Detektion von wenigstens einer Substanz 2, mittels Farbumschlag, insbesondere mittels Fluoreszenz-In-Situ-Hybridisierung (FISH) und/oder mittels Immunassays, wie es hierin beschrieben und/oder beansprucht ist. Insbesondere eignet sich der hierin beschriebene und/oder beanspruchte Sondenkomplex 1 zur Signalverstärkung bei optischem Detektieren mittels Fluoreszenz-In-Situ-Hybridisierung (FISH) und/oder Immunassay und/oder eines anderen Detektionsverfahrens, das auf einer Farbreaktion basiert.

Erfindungsgemäß wird somit vorgeschlagen, einen Sondenkomplex 1 zum spezifischen Nachweis von wenigstens einer Substanz 2 bereitzustellen, wobei der Sondenkomplex 1 an ein Ziel-Segment 7 der Substanz 2 spezifisch anbindet und wobei durch eine Interaktion mit wenigstens einer detektierbaren Markierung 8 ein vorzugsweise optisch nachweisbares Signal auslösbar ist, wobei der Sondenkomplex 1 wenigstens einen Sondentyp als eine Primärsonde 6 mit einem zielspezifischen Segment 5 und wenigstens einen weiteren Sondentyp als eine Sekundärsonde 9 mit einem zielspezifischen Segment 10 aufweist, wobei die zielspezifischen Segmente aller Sondentypen des Sondenkomplexes 1 so aufeinander abgestimmt sind, dass eine Kette 3 von wenigstens drei Sonden 4 bildbar ist.

## Patentansprüche

1. Sondenkomplex (1) zum spezifischen Nachweis von wenigstens einer Substanz (2), wobei der Sondenkomplex (1) an ein Ziel-Segment (7) der Substanz (2) spezifisch anbindet und wobei durch eine Interaktion mit wenigstens einer detektierbaren Markierung (8) ein vorzugsweise optisch nachweisbares Signal auslösbar ist, **dadurch gekennzeichnet, dass** in dem Sondenkomplex (1) wenigstens drei Sondentypen vorhanden sind, wobei der Sondenkomplex (1) wenigstens einen Sondentyp als eine Primärsonde (6) mit einem zielspezifischen Segment (5) aufweist, das zu dem Ziel-Segment (7) der Substanz (2) komplementär ist, und dass der Sondenkomplex (1) wenigstens einen weiteren Sondentyp als eine Sekundärsonde (9) mit einem zielspezifischen Segment (10) aufweist, das zu der wenigstens einen Primärsonde (6) komplementär ist, wobei die zielspezifischen Segmente aller Sondentypen des Sondenkomplexes (1) so aufeinander abgestimmt sind, dass eine Kette (3) von wenigstens drei, insbesondere beliebig vielen, Sonden (4) bildbar ist, sodass eine Kettenlänge gestaltet ist.

2. Sondenkomplex (1) nach Anspruch 1, **dadurch gekennzeichnet, dass** der wenigstens eine Sondentyp eine Nukleinsäure oder ein Antikörper oder ein Peptid ist.

3. Sondenkomplex (1) nach einem der vorangehenden Ansprüche, **dadurch gekennzeichnet, dass** in dem Sondenkomplex (1) wenigstens vier Sondentypen vorhanden sind.

4. Sondenkomplex (1) nach einem der vorangehenden Ansprüche, **dadurch gekennzeichnet, dass** die Kette (3) von Sonden (4) verzweigt oder unverzweigt ausgebildet ist.

5. Sondenkomplex (1) nach einem der vorangehenden Ansprüche, **dadurch gekennzeichnet, dass** in dem Sondenkomplex (1) wenigstens ein weiterer Sondentyp als eine Tertiärsonde (11) mit einem zielspezifischen Segment (12) ausgebildet ist, das zu der wenigstens einen Sekundärsonde (9) komplementär ist, insbesondere mit wenigstens einem weiteren Sondentyp als eine Quartärsonde (13) mit einem zielspezifischen Segment (14), das zu der wenigstens einen Tertiärsonde (11) komplementär ist, und/oder mit einem zielspezifischen Segment (15), das zu der wenigstens einen Sekundärsonde (9) komplementär ist, so dass eine verzweigte oder unverzweigte Kettenstruktur zwischen dem Ziel-Segment (7) der Substanz (2) und dem Sondenkomplex (1) ausgebildet wird.

6. Sondenkomplex (1) nach einem der vorangehenden Ansprüche, **dadurch gekennzeichnet, dass** die Sondentypen jeweils als lineare Sonden und/oder Sonden mit Sekundärstruktur, vorzugsweise Molecular Beacons, ausgebildet sind.

7. Sondenkomplex (1) nach einem der vorangehenden Ansprüche, **dadurch gekennzeichnet, dass** die wenigstens eine detektierbare Markierung (8) an der Primärsonde und/oder dem wenigstens einen weiteren Sondentyp ausgebildet ist und/oder ein Enzymmarker, Affinitätsmarker und/oder ein Farbstoff ist, insbesondere wobei der Farbstoff ein Fluoreszenzfarbstoff ist und/oder der Affinitätsmarker Affinitätsbindungspaare Biotin-Streptavidin oder Antigen-Antikörper umfasst.

8. Sondenkomplex (1) nach einem der vorangehenden Ansprüche, **dadurch gekennzeichnet, dass** das Ziel-Segment (7) der Substanz (2) wenigstens eine DNA- und/oder RNA-Sequenz, vorzugsweise mRNA-Sequenz, und/oder eine Aminosäuren-Sequenz umfasst.

9. Sondenkomplex (1) nach einem der vorangehenden Ansprüche, **dadurch gekennzeichnet, dass** die Substanz (2) eine Nukleinsäure und/oder ein Protein, insbesondere ein Antikörper, und/oder ein Virus und/oder eine niedermolekulare Verbindung, insbesondere ein Hormon, Toxin und/oder Pestizid, umfasst.

10. Verfahren zur Durchführung von Sonden-Kettenreaktion, umfassend die folgenden Schritte: a) Inkontaktbringen einer vorzugsweise in einer Probe enthaltenen Substanz (2) mit wenigstens einem Sondenkomplex (1) nach einem der Ansprüche 1 bis 9; b) Durchführung eines ersten Kreislaufs (16) einer Sonden-Kettenreaktion, wobei wenigstens eine Primärsonde (6) an wenigstens ein Ziel-Segment (7) der Substanz (2) bindet und wenigstens eine Sekundärsonde (9) an die wenigstens eine Primärsonde (6) bindet, insbesondere wobei wenigstens eine Tertiärsonde (11) an die wenigstens eine Sekundärsonde (9) bindet und wenigstens eine Quartärsonde (13) an die wenigstens eine Tertiärsonde (11) und/oder die wenigstens eine Sekundärsonde (9) bindet; c) optional Durchführung eines zweiten Kreislaufs (16) einer Sonden-Kettenreaktion, wobei wenigstens eine weitere Sonde (4) an wenigstens eine der Sekundärsonde, Tertiärsonde und/oder Quartärsonde bindet; und d) vorzugsweise optisches Detektieren der in den einzelnen Substanzen erzeugten Signale.

11. Verfahren nach Anspruch 10, **dadurch gekennzeichnet, dass** die Durchführung der Sonden-Kettenreaktion oder der Sonden-Kettenreaktion unter Verwendung des Sondenkomplexes (1) mittels Farbumschlag, insbesondere mittels Fluoreszenz-In-Situ-Hybridisierung (FISH) und/oder Immunassays, erfolgt.

12. Testkit zur Durchführung des Verfahrens nach einem der Ansprüche 10 bis 11, umfassend wenigstens einen Sondenkomplex nach einem der Ansprüche 1 bis 9 und vorzugsweise wenigstens ein Reagenz mit Enzym.

13. Verwendung des Sondenkomplexes (1) nach einem der Ansprüche 1 bis 9 und/oder eines Verfahrens nach einem der Ansprüche 10 bis 11 und/oder eines Testkits nach Anspruch 12 zum spezifischen Nachweis von Mikroorganismen und/oder deren Eigenschaften.

14. Verwendung des Sondenkomplexes (1) nach Anspruch 13 zur Signalverstärkung bei optischem Detektieren mittels Fluoreszenz-In-Situ-Hybridisierung (FISH) und/oder Immunassay bei dem wenigstens ein Sondentyp vorhanden ist, **dadurch gekennzeichnet, dass** wenigstens zwei Sondentypen so aneinander binden, dass eine Kette (3) von Sonden (4) gebildet ist.

## Claims

1. Probe complex (1) for the specific detection of at least one substance (2), wherein the probe complex (1) specifically binds to a target segment (7) of the substance (2) and wherein an interaction with at least one detectable label (8) triggers a signal that can preferably be detected optically, **characterized in that** at least three probe types are present in the probe complex (1), wherein the probe complex (1) has at least one probe type as a primary probe (6) with a target-specific segment (5) that is complementary to the target segment (7) of the substance (2), and **in that** the probe complex (1) has at least one further probe type as a secondary probe (9) with a target-specific segment (10) that is complementary to the at least one primary probe (6), wherein the target-specific segments of all probe types of the probe complex (1) are coordinated with each other in such a way that a chain (3) of at least three, in particular any number of probes (4) can be formed, so that a chain length is created.

2. Probe complex (1) according to claim 1, **characterized in that** the at least one probe type is a nucleic acid or an antibody or a peptide.

3. Probe complex (1) according to one of the preceding claims, **characterized in that** at least four probe types are present in the probe complex (1).

4. Probe complex (1) according to one of the preceding claims, **characterized in that** the chain (3) of probes (4) is of branched or unbranched design.

5. Probe complex (1) according to one of the preceding claims, **characterized in that** in the probe complex (1) at least one further probe type is formed as a tertiary probe (11) with a target-specific segment (12) that is complementary to the at least one secondary probe (9), in particular with at least one further probe type as a quaternary probe (13) with a target-specific segment (14) which is complementary to the at least one tertiary probe (11), and/or with a target-specific segment (15) which is complementary to the at least one secondary probe (9), so that a branched or unbranched chain structure is formed between the target segment (7) of the substance (2) and the probe complex (1).

6. Probe complex (1) according to one of the preceding claims, **characterized in that** the probe types are each designed as linear probes and/or probes with a secondary structure, preferably molecular beacons.

7. Probe complex (1) according to one of the preceding claims, **characterized in that** the at least one detectable label (8) is formed on the primary probe and/or the at least one further probe type and/or is an enzyme marker, affinity marker and/or a dye, in particular wherein the dye is a fluorescent dye and/or the affinity marker comprises biotin-streptavidin or antigen-antibody affinity binding pairs.

8. Probe complex (1) according to one of the preceding claims, **characterized in that** the target segment (7) of the substance (2) comprises at least one DNA and/or RNA sequence, preferably mRNA sequence, and/or an amino acid sequence.

9. Probe complex (1) according to one of the preceding claims, **characterized in that** the substance (2) comprises a nucleic acid and/or a protein, in particular an antibody, and/or a virus and/or a low molecular weight compound, in particular a hormone, toxin and/or pesticide.

10. Method for carrying out a probe chain reaction, comprising the following steps:
a) bringing a substance (2), preferably contained in a sample, into contact with at least one probe complex (1) according to one of claims 1 to 9; b) carrying out a first cycle (16) of a probe chain reaction, wherein at least one primary probe (6) binds to at least one target segment (7) of the substance (2) and at least one secondary probe (9) binds to the at least one primary probe (6), in particular wherein at least one tertiary probe (11) binds to the at least one secondary probe (9) and at least one quaternary probe (13) binds to the at least one tertiary probe (11) and/or the at least one secondary probe (9); c) optionally carrying out a second cycle (16) of a probe chain reaction, wherein at least one further probe (4) binds to at least one of the secondary probe, tertiary probe, and/or quaternary probe;
and d) preferably optically detecting the signals generated in the individual substances.

11. Method according to claim 10, **characterized in that** the probe chain reaction or the probe chain reaction using the probe complex (1) is carried out by means of color change, in particular by means of fluorescence in situ hybridization (FISH) and/or immunoassays.

12. Test kit for carrying out the method according to one of claims 10 to 11, comprising at least one probe complex according to one of claims 1 to 9 and preferably at least one reagent with enzyme.

13. Use of the probe complex (1) according to one of claims 1 to 9 and/or a method according to one of claims 10 to 11 and/or a test kit according to claim 12 for the specific detection of microorganisms and/or their properties.

14. Use of the probe complex (1) according to claim 13 for signal amplification in optical detection by means of fluorescence in situ hybridization (FISH) and/or immunoassay in which at least one probe type is present, **characterized in that** at least three probe types bind to each other in such a way that a chain (3) of probes (4) is formed.

## Revendications

1. Complexe de sonde (1) permettant de déceler de manière spécifique la présence d'au moins une substance (2), dans lequel le complexe de sonde (1) se lie spécifiquement à un segment cible (7) de la substance (2) et dans lequel une interaction avec au moins un marqueur détectable (8) permet de déclencher un signal décelable de préférence de manière optique, **caractérisé en ce que**, dans le complexe de sonde (1), sont présents au moins trois types de sonde, dans lequel le complexe de sonde (1) présente au moins un type de sonde servant de sonde primaire (6) avec un segment spécifique de cible (5) qui est complémentaire au segment cible (7) de la substance (2), et **en ce que** le complexe de sonde (1) présente au moins un autre type de sonde servant de sonde secondaire (9) avec un segment spécifique de cible (10) qui est complémentaire à l'au moins une sonde primaire (6), dans lequel les segments spécifiques de cible de tous les types de sonde du complexe de sonde (1) sont coordonnés les uns aux autres de telle sorte qu'une chaîne (3) d'au moins trois sondes (4), en particulier une quantité quelconque, peut être formée de manière à façonner une longueur de chaîne.

2. Complexe de sonde (1) selon la revendication 1, **caractérisé en ce que** l'au moins un type de sonde est un acide nucléique ou un anticorps ou un peptide.

3. Complexe de sonde (1) selon l'une des revendications précédentes, **caractérisé en ce que**, dans le complexe de sonde (1), sont présents au moins quatre types de sonde.

4. Complexe de sonde (1) selon l'une des revendications précédentes, **caractérisé en ce que** la chaîne (3) de sondes (4) est conçue de manière ramifiée ou non ramifiée.

5. Complexe de sonde (1) selon l'une des revendications précédentes, **caractérisé en ce que**, dans le complexe de sonde (1), au moins un autre type de sonde servant de sonde tertiaire (11) est conçu avec un segment spécifique de cible (12) qui est complémentaire à l'au moins une sonde secondaire (9), en particulier avec au moins un autre type de sonde servant de sonde quaternaire (13) avec un segment spécifique de cible (14) qui est complémentaire à l'au moins une sonde tertiaire (11) et/ou avec un segment spécifique de cible (15) qui est complémentaire à l'au moins une sonde secondaire (9) de telle sorte qu'une structure de chaîne ramifiée ou non ramifiée entre le segment cible (7) de la substance (2) et le complexe de sonde (1) soit conçue.

6. Complexe de sonde (1) selon l'une des revendications précédentes, **caractérisé en ce que** les types de sonde sont à chaque fois conçus sous forme de sondes linéaires et/ou de sondes avec une structure secondaire, de préférence des balises moléculaires.

7. Complexe de sonde (1) selon l'une des revendications précédentes, **caractérisé en ce que** l'au moins un marqueur détectable (8) est conçu sur la sonde primaire et/ou l'au moins un autre type de sonde et/ou est un marqueur enzymatique, un marqueur d'affinité et/ou un colorant, en particulier dans lequel le colorant est un colorant fluorescent et/ou le marqueur d'affinité comprend des paires de liaisons d'affinité biotine-streptavidine ou antigène-anticorps.

8. Complexe de sonde (1) selon l'une des revendications précédentes, **caractérisé en ce que** le segment cible (7) de la substance (2) comprend au moins une séquence d'ADN et/ou une séquence d'ARN, de préférence une séquence d'ARNm, et/ou une séquence d'acides aminés.

9. Complexe de sonde (1) selon l'une des revendications précédentes, **caractérisé en ce que** la substance (2) comprend un acide nucléique et/ou une protéine, en particulier un anticorps et/ou un virus et/ou un composé de faible poids moléculaire, en particulier une hormone, une toxine et/ou un pesticide.

10. Procédé permettant de réaliser une réaction en chaîne de sondes, comprenant les étapes suivantes : a) mettre en contact une substance (2), de préférence contenue dans un échantillon, avec au moins un complexe de sonde (1) selon l'une des revendications 1 à 9 ; b) réaliser un premier cycle (16) d'une réaction en chaîne de sondes, dans lequel au moins une sonde primaire (6) se lie à au moins un segment cible (7) de la substance (2) et au moins une sonde secondaire (9) se lie à l'au moins une sonde primaire (6), en particulier dans lequel au moins une sonde tertiaire (11) se lie à l'au moins une sonde secondaire (9) et au moins une sonde quaternaire (13) se lie à l'au moins une sonde tertiaire (11) et/ou l'au moins une sonde secondaire (9) ; c) réaliser en option un second cycle (16) d'une réaction en chaîne de sondes, dans lequel au moins une autre sonde (4) se lie à au moins une des sondes parmi la sonde secondaire, la sonde tertiaire et/ou la sonde quaternaire ; et d) détecter de préférence de manière optique les signaux produits dans les substances individuelles.

11. Procédé selon la revendication 10, **caractérisé en ce que** la réalisation de la réaction en chaîne de sondes ou de la réaction en chaîne de sondes en utilisant le complexe de sonde (1) se fait à l'aide d'un changement de couleur, en particulier à l'aide d'une hybridation *in situ* par fluorescence (FISH) et/ou des immunoessais.

12. Kit de test permettant de réaliser le procédé selon l'une des revendications 10 à 11, comprenant au moins un complexe de sonde selon l'une des revendications 1 à 9 et de préférence au moins un réactif avec enzyme.

13. Utilisation du complexe de sonde (1) selon l'une des revendications 1 à 9 et/ou d'un procédé selon l'une des revendications 10 à 11 et/ou d'un kit de test selon la revendication 12 permettant de déceler de manière spécifique des microorganismes et/ou leurs propriétés.

14. Utilisation du complexe de sonde (1) selon la revendication 13 permettant d'amplifier le signal dans la détection optique à l'aide d'une hybridation *in situ* par fluorescence (FISH) et/ou d'un immunoessai dans lequel au moins un type de sonde est présent, **caractérisé en ce qu'**au moins trois types de sonde se lient les unes aux autres de manière à former une chaîne (3) de sondes (4).
